# EUROPEAN PATENT SPECIFICATION

(11) **EP 1 925 300 B1**
(45) Date of publication and mention of the grant of the patent: **17.11.2010**
(21) Application number: 07022494.4
(22) Date of filing: 20.11.2007
(51) Int. Cl.: A61K 9/70

(54) **Adhesive preparation**
Klebstoffzubereitung
Préparation adhésive

(30) Priority: 22.11.2006 JP 2006315751
(43) Date of publication of application: 28.05.2008
(73) Proprietor: NITTO DENKO CORPORATION, Osaka (JP)
(72) Inventor: Iwao, Yoshihiro, Ibaraki-shi Osaka (JP); Okubo, Katsuyuki, Ibaraki-shi Osaka (JP); Okada, Katsuhiro, Ibaraki-shi Osaka (JP); Matsuoka, Kensuke, Ibaraki-shi Osaka (JP)
(74) Representative: Grünecker, Kinkeldey, Stockmair & Schwanhäusser Anwaltssozietät

(56) References cited:
- EP-A- 0 569 862
- EP-A- 1 270 205
- EP-A- 1 462 121
- EP-A- 1 736 146

## Description

The present invention relates to an adhesive preparation including a backing and a pressure-sensitive adhesive layer which contains a drug and is laminated on at least one side of the backing.

An adhesive preparation for application to a surface of the skin is used in order to percutaneously administer a drug to the body. In the development of adhesive preparations, handleability in application, compliance to skin surface movements (flexibility), no irritation to the skin and the like are highly required, because the application surfaces are skin surfaces.

Adhesive preparations are further required to have properties such as the stability of the whole pressure-sensitive adhesive layer and of the drug itself over a prolonged period (storage stability) and sufficient anchoring of the pressure-sensitive adhesive layer to the backing (anchoring effect).

Japanese Patent No. 2688778 discloses an adhesive patch for disease remedy which comprises: a backing formed by laminating a nonwoven fabric to a base constituted of a film having no openings; and a drug-containing layer having pressure-sensitive adhesiveness at ordinary temperatures and laminated on the nonwoven-fabric side of the backing. There is a statement to the effect that the backing is obtained as follows: a nonwoven fabric is laminated on a film; a hot-melt adhesive sheet/film is interposed between a film and a nonwoven fabric and this laminate is heat-bound; or a nonwoven fabric is laminated on a film with an adhesive binder being interposed therebetween. There is a statement therein to the effect that in this adhesive patch, the drug-containing layer having pressure-sensitive adhesive properties at ordinary temperatures shows improved anchoring to the backing due to the nonwoven fabric.

Japanese Patent No. 3081858 discloses an adhesive patch which comprises: a backing obtained by laminating a polyester film having a thickness of 0.5 to 6 µm to a polyester nonwoven fabric having a basis weight of 5 to 20 g/m²; and a pressure-sensitive adhesive layer laminated on the nonwoven-fabric side of the backing. There is a statement therein to the effect that in laminating the polyester film to the polyester nonwoven fabric, any desired adhesive is applied, for example, to the film side in a given dry amount. There is a statement therein to the effect that this adhesive patch is improved also in anchoring as in the adhesive patch disclosed in Japanese Patent No. 2688778 mentioned above and that the adhesive patch has moderate self-supporting properties owing to the constitution of the backing and has excellent handleability in this respect.

However, since a film and a nonwoven fabric are interposed with a material such as an adhesive, a hot-melt adhesive sheet and the like (hereinafter collectively referred to as "binder") in these adhesive patches, the flexibility or compliance of the patches to the skin may be impaired due to the rigidity of the binder. This tendency is significantly found especially in an adhesive patch using a thin film as its backing. Furthermore, those adhesive patches, during wear on the skin, give an uncomfortable feeling (stiff feeling) to the skin because of the increase in backing thickness due to the thickness of the binder itself. In addition, since the backing has an increased edge thickness, there is a possibility that contacts of the edges to the skin might irritate the skin during wear in some cases.

Furthermore, there is a possibility that some drugs might react with the binder to deteriorate the binder and thereby destroy the bond of the film to the nonwoven fabric, resulting in separation between them. There also is a fear that some drugs themselves may be deteriorated. Thus, in order to apply these adhesive patches to commercial products of adhesive preparations, further research on constituting backings, selecting drugs or the like will be required.

In addition, in the case where a step in which a pressure-sensitive adhesive solution containing an organic solvent is directly applied to a nonwoven fabric in producing an adhesive patch, there is a possibility that the organic solvent contained in the pressure-sensitive adhesive solution which has passed through the nonwoven fabric might deteriorate the binder to thereby destroy the bond between the film and the nonwoven fabric and cause separation between them. To avoid this problem, for example, an adhesive solution containing an organic solution is applied onto a liner prepared otherwise and dried to form a pressure sensitive adhesive layer, and then the resultant layer is transferred to a backing. This method, however, is undesirable because the steps are complicated and the liner, which is unnecessary in the product, should be separately prepared. Consequently, the adhesive patches described above have a low degree of freedom of step selection and there is room for improvement in this respect.

Japanese Patent No. 2939123 relating to a pressure-sensitive adhesive sheet describes that a nonwoven fabric of a thermoplastic resin may be laminated on one side of a backing opposite to the drug-containing adhesive layer. However, this patent does not describe how to laminate the nonwoven fabric.

As mentioned above, an adhesive preparation including a backing in which a film and a nonwoven fabric are directly bound to each other has not yet disclosed at all.

EP-A-1 270 205 discloses an adhesive sheet comprising a substrate comprising a synthetic resin film and a nonwoven fabric laminated on one side of the film, and an adhesive layer formed on the nonwoven fabric, wherein the nonwoven fabric comprises polyethylene imide at least on one surface of the nonwoven fabric.

EP-A-0 569 862 discloses a medical adhesive sheet comprising a support comprising a laminate of a polyester film having a thickness of from 0.5 to 6 µm and a polyester nonwoven fabric having a basic weight of 5 to 20 g/m² and a pressure-sensitive adhesive layer laminated on the surface of the nonwoven fabric.

EP-A-1 736 146 discloses a nicotine transdermal delivery system comprising an adhesive layer comprising a free-based nicotine and a liquid ingredient compatible with the adhesive, wherein the adhesive layer is crosslinked, and the liquid ingredient is contained in a proportion of 20 to 75 parts by weight per 100 parts by weight of the adhesive layer as a whole.

In view of the above, an object of the invention is to provide an adhesive preparation which is flexible enough to comply to the skin and has reduced skin irritation and high stability.

Accordingly, the invention relates to an
adhesive preparation comprising:
   a backing; and
   a pressure-sensitive adhesive layer which contains a drug and is disposed on at least one side of the backing,
   wherein the backing comprises a polyester film having a thickness of 0.5 to 6.0 µm, and a polyester nonwoven fabric directly bound to said film without using a binder,
wherein the drug has at least one primary to tertiary amine structure within the molecule thereof.
Preferred embodiments are set forth in the subclaims.

According to the adhesive preparation of the invention, the backing includes a polyester film having a thickness of 0.5 to 6.0 µm, and a polyester nonwoven fabric which is directly bound to the polyester film without using a binder. According to the adhesive preparation of the invention, the film itself in the backing is thin and the adhesive preparation is not always influenced by the rigidity of a binder. Consequently, the adhesive preparation of the invention has satisfactory flexibility and compliance to skin surface movements.

In addition to the thin thickness of the film itself, the adhesive preparation of the invention does not necessarily require a binder resulting in no thickness of such a binder. This enables a thin thickness of the adhesive preparation and this adhesive preparation is less apt to give an uncomfortable feeling (stiff feeling) to the skin. In addition, this adhesive preparation is advantageous in low skin irritation caused by the contact of the backing edge to the skin during its wear on the skin, owing to the thin thickness of the backing edge.

In the case where the pressure-sensitive adhesive layer is laminated on the nonwoven-fabric side of the backing, the pressure-sensitive adhesive layer intertwines with polyester fibers of the nonwoven fabric in the backing. Consequently, the pressure-sensitive adhesive layer shows excellent anchoring to the backing.

Furthermore, in the case where the pressure-sensitive adhesive layer is laminated on the nonwoven-fabric side of the backing, directly binding the nonwoven fabric to the film without the necessity of a binder according to the invention may avoid the binder deterioration due to the reaction of the drug with the binder. This may also avoid separation of the film from the nonwoven fabric. Likewise, drug deterioration due to reaction of the drug with the binder may also be avoided. Thus, various ranges of drugs are practically applicable to the highly valued adhesive preparation according to the invention in which the pressure sensitive adhesive layer is laminated on the nonwoven fabric side of the backing and no binder is used for binding the nonwoven fabric to the film.

Furthermore, in the case where the pressure-sensitive adhesive layer is laminated on the nonwoven-fabric side of the backing, the adhesive preparation without using a binder according to the invention may avoid binder deterioration due to an organic solvent in the pressure sensitive adhesive solution passed through the nonwoven fabric and therefore may avoid separation of the both, even when a step in which a pressure-sensitive adhesive solution containing an organic solvent is directly applied to a nonwoven fabric is employed. Consequently, in the case where the pressure-sensitive adhesive layer is laminated on the nonwoven-fabric side of the backing, the adhesive preparation of the invention is advantageous because of a high degree of freedom of step selection.

In addition, in the case where the pressure-sensitive adhesive layer is laminated on the nonwoven-fabric side of the backing, the adhesive preparation of the invention has excellent stability because binder deterioration through drug/binder reaction, drug deterioration attributable to a binder, and nonwoven fabric/film separation attributable to binder deterioration can be inhibited.

The adhesive preparation of the invention includes a backing, and a pressure-sensitive adhesive layer which contains a drug and is laminated on at least one side of the backing. The backing includes a polyester film having a thickness of 0.5 to 6.0 µm, and a polyester nonwoven fabric directly bound to the film. As used herein, the terms "polyester film", "polyester nonwoven fabric" and "polyester fiber" mean a film, a nonwoven fabric and a fiber, respectively, each of which contains a polyester as the major ingredient thereof.

In the invention, the thickness of the polyester film is 0.5 to 6.0 µm. From the standpoint of reducing irritation due to the backing edges, the thickness thereof is preferably as small as possible. From the standpoint of practical use, it is preferred to employ an ultrathin film having a thickness of 1 to 5 µm, especially 1.5 to 4.5 µm. When the thickness of the film is smaller than 0.5 µm, this film is impracticable because it is difficult to produce the backing according to the invention by laminating this film to the nonwoven fabric which will be described later. When the thickness thereof exceeds 6 µm, the adhesive preparation comes to have the rigidity of the polyester itself and gives an uncomfortable feeling (stiff feeling) during wear on the skin surface.

The polyester nonwoven fabric to be used in the invention is constituted of polyester fibers arranged irregularly. This fabric is preferred because it has a higher degree of surface irregularities than woven or knit polyester fabrics in which polyester fibers are arranged regularly and, hence, brings about satisfactory anchoring when a pressure-sensitive adhesive layer is formed on the nonwoven-fabric side.

In the invention, the basis weight of the polyester nonwoven fabric is not particularly limited. However, from the standpoint of reducing the uncomfortable feeling which may be given during wear on the skin surface, it is preferred in the invention to employ a polyester nonwoven fabric having a smaller basis weight than those in general use. Such a basis weight is preferably 5 to 40 g/m², more preferably 5 to 25 g/m², even more preferably 5 to 20 g/m², and most preferably 8 to 20 g/m².

When the basis weight thereof is smaller than 5 g/m², there are cases where the pressure-sensitive adhesive layer laminated on the nonwoven-fabric side of the backing does not show sufficiently improved anchoring to the backing. When the basis weight thereof exceeds 40 g/m², there are cases where the adhesive preparation gives an uncomfortable feeling due to this nonwoven fabric during wear on the skin.

Incidentally, in the case where a nonwoven fabric is bound to a film through a binder as in conventional products, a nonwoven fabric which has a reduced basis weight and which itself has a reduced thickness may be used for the purpose of, e.g., improving the flexibility and compliance to the skin of the adhesive preparation. However, the following problem may arise. In this case, the binder applied to the film passes through the nonwoven fabric and reaches the side of the nonwoven fabric which is opposite to the film side. When the backing in which the nonwoven fabric and the film are laminated is wound into a roll, the binder may foul the film side.

Such a problem is eliminated by directly binding the film to the nonwoven fabric without using a binder as in the invention. Accordingly, the invention is especially advantageous when the nonwoven fabric has a small basis weight.

The polyesters to be used for forming the polyester film and polyester nonwoven fabric are not particularly limited. Examples thereof include poly(ethylene terephthalate), poly(butylene terephthalate), poly(ethylene naphthalate), and poly(butylene naphthalate). From the standpoints of safety (nontoxicity) for the living body, suitability for practical use and general use, etc., it is preferred to employ a polyester composed mainly of poly(ethylene terephthalate).

For example, use may be made of an ethylene terephthalate homopolymer, a copolymer containing ethylene terephthalate units as the main units and further containing other ester units, a mixture of an ethylene terephthalate homopolymer and a polymer containing other ester units, or the like.

In forming other ester units in producing such copolymer or mixture, the following dicarboxylic acid ingredients and diol ingredients can, for example, be used. Usable dicarboxylic acid ingredients include aromatic dicarboxylic acids such as isophthalic acid, diphenyldicarboxylic acid, (diphenyl ether)dicarboxylic acid, (diphenyl sulfone)dicarboxylic acid, and naphthalenedicarboxylic acid and aliphatic dicarboxylic acids such as adipic acid and sebacic acid. Usable diol ingredients include alkylene glycols such as trimethylene glycol, tetramethylene glycol, and hexamethylene glycol, aromatic diols such as hydroquinone, resorcinol, and bisphenol A, aliphatic diols such as bis(hydroxyethoxyphenyl) sulfone and bis(hydroxyethoxyphenyl)propane, and diethylene glycol.

The material of the film and the material of the nonwoven fabric may be of the same kind or of different kinds so long as they are polyesters. From the standpoint of enhancing compatibility between the film and the nonwoven fabric and thereby facilitating the direct binding thereof, it is preferred that both materials should be of the same kind. The term "the same kind" herein means that use of the same monomers suffices. Such polyesters, even when differing in the degree of polymerization, are of "the same kind".

In the invention, it is necessary that the nonwoven fabric and the film should be directly bound to each other. The term "directly bound" herein means that the film and the nonwoven fabric are bound to each other in at least a part of the contact interface between them without using a component interposed between them, e.g., a binder. Techniques for directly binding the nonwoven fabric to the film are not particularly limited. For example, the binding is accomplished by fusion-bonding the film to the nonwoven fabric in at least a part of the contact interface between them by means of heat, a solvent, etc. Thermal fusion bonding is preferred from the standpoints of freedom from a danger from the handling of an organic solvent and of convenience.

Whether a nonwoven fabric and a film are directly bound to each other or not is judged based on the following criterion. Namely, when at least a part of the contact parts between the film and the nonwoven fabric in the backing is examined by instrumental analysis or the like and no ingredient other than the film and nonwoven fabric is detected, it can be judged that the nonwoven fabric and the film are directly bound to each other.

Incidentally, the direct bond of the polyester film to the polyester nonwoven fabric is expected to produce the effect of improving the transparency of the backing. This is presumed that the partial melting of the polyester fibers in the nonwoven fabric results in a decrease in the surface area of the fibers in the nonwoven fabric to thereby reduce light scattering. In such a case, as a result of the improvement on the backing transparency, the effect of facilitating the detection of foreign matters in adhesive preparations in the course of adhesive preparation production can be also expected.

In the invention, polyester fibers in the nonwoven fabric may be directly bound to preferably a part of the polyester film to which the nonwoven fabric is bound. Namely, when the binding parts of the nonwoven fabric where polyester fibers are bound to the film are projected on the film surface, then the projected area of the binding parts may be a part of the area of the nonwoven fabric projected on the film surface in the case that the nonwaven fabric is projected on the film surface.

In the case where polyester fibers in the nonwoven fabric are thus bound directly to a part of the polyester film to which the nonwoven fabric is bound, a given proportion of the polyester fibers of the nonwoven fabric are not bound to the film and are hence capable of freely moving in some degree. Such a backing is more flexible than a backing in which the nonwoven fabric is bound to the film through a binder. The poor flexibility of the backing in which the nonwoven fabric is bound (adhered) to the film through a binder is thought to be attributable to that property of the binder by which the binder during adhesion has flowability and hence spreads almost over the whole contact interface between the nonwoven fabric and the binder during the adhesion of the nonwoven fabric to the film. This backing in which the nonwoven fabric is bound (adhered) to the film through a binder is in a state in which polyester fibers in the nonwoven fabric are bound through the binder to the polyester film substantially in all region of the nonwoven fabric. It is presumed that such a backing accordingly has a smaller proportion of those polyester fibers of the nonwoven fabric which are freely movable against the film and, hence, has poor flexibility.

From such a standpoint, the binding proportion between the polyester fibers of the nonwoven fabric and the film [100×(area of the binding parts between the nonwoven-fabric polyester fibers and the film which are projected on the film surface in the case that the binding parts are projected on the film surface)/(area of the nonwoven fabric projected on the film surface in the case that the nonwoven fabric is projected on the film surface)] is preferably 10 to 80%, more preferably 20 to 70%. When the proportion thereof is lower than 10%, there is a fear that the binding strength between the film and the nonwoven fabric may be insufficient. When the proportion thereof exceeds 80%, there is a possibility that the backing might give a stiff feeling during wear on the skin.

The area of the binding parts between the nonwoven-fabric polyester fibers and the film which are projected on the film surface is a value measured in the following manner. In the case where nonwoven-fabric fibers fused to the film are difficult to observe in an examination from the nonwoven-fabric side with an optical microscope or electron microscope, the following procedure is conducted. The nonwoven fabric bound to the film is forcedly peeled off with a commercial pressure-sensitive adhesive tape or by picking, and the film is examined with an optical microscope or electron microscope for binding marks left thereon. The area of the binding marks is calculated through image analysis by binarization. Alternatively, in the case where nonwoven-fabric fibers bound to the film are observed in an examination from the nonwoven-fabric side with an optical microscope or electron microscope, the area occupied by the parts of the bound nonwoven-fabric fibers is calculated through image analysis by binarization.

In one embodiment of the invention, the pressure-sensitive adhesive layer is directly or indirectly laminated on the nonwoven-fabric side of the backing. In this embodiment, the pressure-sensitive adhesive layer shows satisfactory anchoring to the backing as briefly stated hereinabove. This embodiment has advantages, for example, that the adhesive preparation has excellent stability because binder deterioration through drug/binder reaction, drug deterioration attributable to a binder, and nonwoven fabric/film separation attributable to binder deterioration can be sufficiently inhibited.

The term "laminated directly herein means that the pressure-sensitive adhesive layer is laminated on the nonwoven-fabric side without interposing a component between the pressure-sensitive adhesive layer and the nonwoven fabric. The term "laminated indirectly" means that the pressure-sensitive adhesive layer is laminated on the nonwoven-fabric side through a component interposed between the pressure-sensitive adhesive layer and the nonwoven fabric, for example, through some kind of layer such as an undercoat layer. The undercoat layer preferably is a layer which enhances anchoring between the pressure-sensitive adhesive layer and the nonwoven fabric.

Examples of the layer which enhances anchoring between the pressure-sensitive adhesive layer and the nonwoven fabric include a layer containing an ethyleneimine-modified acrylic polymer and a layer containing polyethyleneimine. Such layers are preferred because they bring about satisfactory anchoring properties. The reasons for this are presumed to be as follows. Active amino groups in the layer containing an ethyleneimine-modified acrylic polymer or polyethyleneimine form chemical bonds, such as ionic bonds or amide bonds, with functional groups in the pressure-sensitive adhesive layer, such as carboxyl groups. As a result, anchoring between the pressure-sensitive adhesive layer and the nonwoven fabric is enhanced.

Such an ethyleneimine-modified acrylic polymer is an acrylic polymer modified by the addition of units formed by the ring-opening reaction of ethyleneimine to the acrylic-polymer molecule. It can be obtained by reacting ethyleneimine simultaneously with the preparation of an acrylic polymer or by preparing an acrylic polymer beforehand and modifying the polymer by reaction with ethyleneimine. The polyethyleneimine is a polymer having repeating units represented by -CH₂CH₂NH-.

Incidentally, in the case where a film and a nonwoven fabric are bound to each other through a binder, the proportion of nonwoven-fabric fibers freely movable against the film tends to be small as stated hereinabove. As a result, there are cases where, when a pressure-sensitive adhesive layer is formed on the nonwoven-fabric side of the backing as in this embodiment, then it is difficult to obtain anchoring between the nonwoven fabric and the pressure-sensitive adhesive layer in a short time period or the resultant adhesive preparation has a deficiency in anchoring. On the other hand, in case where the amount of the binder is reduced in order to increase the proportion of freely movable nonwoven-fabric fibers in expectation of such anchoring, it becomes difficult to obtain a sufficient binding strength between the nonwoven fabric and the film.

In contrast thereto, especially in the embodiment of the invention in which polyester fibers in the nonwoven fabric are directly bound to a part of the polyester film to which the nonwoven fabric is bound, the backing has nonwoven-fabric fibers freely movable against the film. Accordingly, when a pressure-sensitive adhesive layer is laminated on the nonwoven-fabric side of this backing, the sufficient anchoring of the pressure-sensitive adhesive layer to the backing can be secured in a short time period in adhesive preparation production. Adhesive preparations can hence be efficiently produced.

Methods for laminating a pressure-sensitive adhesive layer on the nonwoven-fabric side of the backing are not particularly limited. For example, use may be made of a method in which a pressure-sensitive adhesive solution is directly applied to the nonwoven-fabric side of the backing and dried to form a pressure-sensitive adhesive layer (direct formation), or a method in which a pressure-sensitive adhesive solution is applied to a separately prepared liner and dried to form a pressure-sensitive adhesive layer and this pressure-sensitive adhesive layer is then transferred to the nonwoven-fabric side of the backing.

The pressure-sensitive adhesive to be used for forming the pressure-sensitive adhesive layer is not particularly limited. Examples thereof include acrylic pressure-sensitive adhesives containing an acrylic polymer; rubber-based pressure-sensitive adhesives such as styrene/isoprene/styrene block copolymers, styrene/butadiene/styrene block copolymers, polyisoprene, polyisobutylene, and polybutadiene; silicone type pressure-sensitive adhesives such as silicone rubbers, dimethylsiloxane-based polymers, and diphenylsiloxane-based polymers; vinyl ether type pressure-sensitive adhesives such as poly(vinyl methyl ether), poly(vinyl ethyl ether), and poly(vinyl isobutyl ether); vinyl ester type pressure-sensitive adhesives such as vinyl acetate/ethylene copolymers; and polyester type pressure-sensitive adhesives produced from a carboxylic acid ingredient, e.g., dimethyl terephthalate, dimethyl isophthalate, or dimethyl phthalate, and a polyhydric alcohol ingredient, e.g., ethylene glycol. One pressure-sensitive adhesive may be used, or a combination of two or more pressure-sensitive adhesives may be used.

Preferred of those pressure-sensitive adhesives are acrylic pressure-sensitive adhesives. This is because acrylic pressure-sensitive adhesives can be easily crosslinked and give a pressure-sensitive adhesive layer which can hold a large amount of a liquid ingredient therein and hence give a soft feeling during wear on the skin. Examples of such acrylic pressure-sensitive adhesives include acrylic ester type pressure-sensitive adhesives containing, as the main component, a polymer obtained by polymerizing one or more C₂₋₁₈ alkyl esters of (meth)acrylic acid as a monomer ingredient. From the standpoint of satisfactory adhesion to the human skin, acrylic ester type pressure-sensitive adhesives produced using acrylic acid as a copolymerizing ingredient are preferred. From the standpoint of ease of repetitions of application/stripping, a pressure-sensitive adhesive obtained by copolymerizing 2-ethylhexyl acrylate as an alkyl ester of (meth)acrylic acid, acrylic acid, and N-vinyl-2-pyrrolidone in a weight ratio of (40-99.9):(0.1-10):(0-50) is preferred.

On the other hand, from the standpoint of drug stability, rubber-based pressure-sensitive adhesives are preferred. Examples of such rubber-based pressure-sensitive adhesives include rubber-based pressure-sensitive adhesives containing as the main component at least one member selected from polyisobutylene, polyisoprene, and styrene/diene/styrene copolymers. A pressure-sensitive adhesive containing a blend of a high-molecular polyisobutylene having a viscosity-average molecular weight of 500,000 to 5,500,000 and a low-molecular polyisobutylene having a viscosity-average molecular weight of 10,000 to 200,000 in a weight ratio of from 95:5 to 5:95 is preferred because this pressure-sensitive adhesive attains high drug stability and can combine necessary adhesive force and cohesive force. A tackifier may be added according to the necessity.

The thickness of the pressure-sensitive adhesive layer is not particularly limited. However, it is generally 10 to 200 µm, preferably 15 to 150 µm.

According to the necessity, an organic liquid ingredient compatible with the pressure-sensitive adhesive ingredient may be incorporated into the pressure-sensitive adhesive layer in an amount of about 0.1 to 60% by weight, preferably about 30 to 50% by weight, in order to enable the pressure-sensitive adhesive layer to give a soft feeling and have reduced irritating properties during wear on the skin surface. According to the necessity, a crosslinking treatment may be conducted in forming a pressure-sensitive adhesive layer.

Examples of the organic liquid ingredient include glycols such as ethylene glycol, diethylene glycol, propylene glycol, triethylene glycol, polyethylene glycol, and polypropylene glycol; fats and oils such as olive oil, caster oil, squalane, and lanolin; hydrocarbons such as liquid paraffin; various surfactants; ethoxy stearyl alcohol; glycerol monoesters such as oleic acid monoglyceride, caprylic acid monoglyceride, and lauric acid monoglyceride; glycerol diesters, glycerol triesters, and mixtures thereof; alkyl esters of fatty acids such as ethyl laurate, isopropyl myristate, isotridecyl myristate, octyl palmitate, isopropyl palmitate, ethyl oleate, and diisopropyl adipate; fatty acids such as oleic acid and caprylic acid; and other compounds including N-methylpyrrolidone and 1,3-butanediol.

Examples of the crosslinking treatment include physical crosslinking by irradiation with a radiation such as ultraviolet irradiation or electron beam irradiation; and chemical crosslinking treatments with various crosslinking agents such as isocyanate compounds, e.g., trifunctional isocyanates, organic peroxides, organometal salts, metal alcoholates, metal chelate compounds, and polyfunctional compounds (polyfunctional external crosslinking agents and polyfunctional monomers for internal crosslinking such as diacrylates and dimethacrylates.)

The drug contained in the pressure-sensitive adhesive layer is not particularly limited. However, one having percutaneous permeability is preferred. Either a local drug or a systemic drug may be used. Specifically, usable drugs include corticosteroids, analgesic anti-inflammatory agents, hypnotic sedatives, tranquilizing agents, antihypertensive agents, antihypertensive diuretics, antibiotics, anesthetic agents, antimicrobials, antifungal agents, vitamin preparations, coronary vasodilators, antihistamine agents, cough medicines, sexual hormones, antidepressant agents, cerebral vasodilators, antiemetic agents, antitumor agents, and biomedicines. A combination of two or more of these drugs can be incorporated according to the necessity.

In the invention the drug has at least one primary to tertiary amine structure within the drug molecule. The reasons for this are as follows. A polyester resin having a low degree of polymerization is often used as a binder between a polyester film and a polyester nonwoven fabric from the standpoint of adhesion between the film and the nonwoven fabric. There are cases where such a binder is slightly acidic because it contains a large amount of carboxyl groups at ends of the binder molecule chains. Because of this, a drug having an amine moiety is apt to migrate to the acidic binder due to the basicity of the amine moiety. As a result, the binder is apt to be deteriorated by the drug, and separation between the film and the nonwoven fabric is apt to occur. There also is a possibility that drug deterioration might occur simultaneously. Consequently, the invention in which the pressure-sensitive adhesive layer contains a drug having at least one primary to tertiary amine structure is especially advantageous because the backing and the drug have high stability since the film and the nonwoven fabric are directly bound to each other without using a binder.

The content of such a drug can be suitably determined according to the kind of the drug and the purpose of administration. In general, however, a drug is incorporated into the pressure-sensitive adhesive layer in an amount of 0.5 to 40% by weight, preferably 1 to 30% by weight. When the content thereof is lower than 0.5% by weight, there is a possibility that drug release in an amount effective in a remedy cannot be expected. When the content thereof exceeds 40% by weight, there is a possibility that the remedial effect comes to have a limitation and such a large drug amount is economically disadvantageous.

In the invention, it is also possible to laminate the pressure-sensitive adhesive layer not on the nonwoven-fabric side of the backing but on the film side thereof. This embodiments has the following advantage. When this adhesive preparation is enclosed and packed in a packaging material, the nonwoven fabric of the adhesive preparation comes into contact with the inner surface of the packaging material. The area of substantial contact with the adhesive preparation hence decreases and the force of friction between them decreases accordingly. As a result, the adhesive preparation can be easily taken out of the packaging material.

### Examples

The invention will be further explained by reference to the following specific Examples. Hereinafter, "parts" means "parts by weight" unless otherwise indicated.

### EXAMPLE 1

In an inert gas atmosphere, 95 parts of 2-ethylhexyl acrylate, 5 parts of acrylic acid, and 0.2 parts of benzoyl peroxide were solution-polymerized at 60°C in ethyl acetate to prepare a solution of an acrylic pressure-sensitive adhesive A. 5.5 parts of the acrylic pressure-sensitive adhesive A on a solid basis, 40 parts of isopropyl myristate, and 5 parts of isosorbide dinitrate were mixed together by stirring in a vessel until the mixture became homogeneous. Ethyl acetoacetate aluminum diisopropylate was added thereto in an amount of 0.165 parts, and the viscosity of the mixture was regulated with ethyl acetate. Thus, a pressure-sensitive adhesive solution was obtained.

The pressure-sensitive adhesive solution was applied to the nonwoven-fabric side of a backing obtained by thermally fusion-bonding a nonwoven fabric made of poly(ethylene terephthalate) (basis weight, 12 g/m²) to a poly(ethylene terephthalate) film (2-µm thick), in such an amount as to result in a thickness after drying of 80 µm. The backing coated was dried at 100°C for 3 minutes in a hot-air circulating drying oven and then subjected to a 24-hour heat treatment. A poly(ethylene terephthalate) liner (75 µm) which had undergone a silicone releasant treatment was press-bound on its releasant side to the surface of the pressure-sensitive adhesive to obtain an adhesive preparation according to the invention.

The pressure-sensitive adhesive layer could be thus directly formed on the backing used above according to the invention.

### COMPARATIVE EXAMPLE 1

A pressure-sensitive adhesive solution obtained in the same manner as in Example 1 was applied to the nonwoven-fabric side of a composite film obtained by laminating a nonwoven fabric made of poly(ethylene terephthalate) (basis weight, 12 g/m²) to a film made of poly(ethylene terephthalate) (2-µm thick) with a polyester type adhesive (application amount, 1-2 g/m²), in such an amount as to result in a thickness after drying of 80 µm. However, destruction occurred at the binding interface between the nonwoven poly(ethylene terephthalate) fabric and the poly(ethylene terephthalate) film to cause partial separation of the film before the coated composite film was introduced into a hot-air circulating drying oven.

### EXAMPLE 2

A pressure-sensitive adhesive solution obtained in the same manner as in Example 1 was applied to a poly(ethylene terephthalate) liner (75 µm) which had undergone a silicone releasant treatment, in such an amount as to result in a thickness after drying of 80 µm. This coated liner was dried at 100°C for 3 minutes in a hot-air circulating drying oven and then subjected to a 24-hour heat treatment. Thus, a pressure-sensitive adhesive layer was obtained. The nonwoven-fabric side of the same backing as in Example 1 was press-bound to the pressure-sensitive adhesive layer with a rubber roller to produce an adhesive preparation according to the invention.

### COMPARATIVE EXAMPLE 2

An adhesive preparation was produced in the same manner as in Example 2, except that the backing used was the same as that used in Comparative Example 1.

### COMPARATIVE EXAMPLE 3

An adhesive preparation was produced in the same manner as in Example 2, except that the backing used was the backing used in Example 1 in which the thickness of the polyethylene terephthalate film was 25 µm.

### EXAMPLE 3

An ethyl acetate solution of the acrylic pressure-sensitive adhesive A containing no drug was prepared according to the following formulation. This pressure-sensitive adhesive solution was applied to a poly(ethylene terephthalate) liner (75 µm) which had undergone a silicone releasant treatment, in such an amount as to result in a thickness after drying of 75 µm. This coated liner was dried at 100°C for 3 minutes in a hot-air circulating drying oven and then subjected to a heat treatment for 24 hours. Thus, a pressure-sensitive adhesive layer was obtained. The same backing as in Example 1 was press-bound on its nonwoven-fabric side to the pressure-sensitive adhesive layer with a rubber roller to produce an adhesive preparation. Furthermore, the liner of this adhesive preparation was stripped off, and nicotine was applied to the surface of the pressure-sensitive adhesive layer with a die coater to absorb the nicotine in such an amount that the pressure-sensitive adhesive layer came to contain 10 parts of nicotine per 60 parts of the acrylic pressure-sensitive adhesive A in the pressure-sensitive adhesive layer. Thus, an adhesive preparation according to the invention was obtained. Incidentally, nicotine is a substance having two tertiary amine structures in the molecule.

### Formulation

| Pressure-sensitive adhesive ingredient: | | |
|---|---|---|
| | Acrylic pressure-sensitive adhesive A | 60 parts |

| Liquid ingredient: | | |
|---|---|---|
| | Isopropyl palmitate | 30 parts |

| Crosslinking agent: | | |
|---|---|---|
| | Ethyl acetoacetate aluminum diisopropylate | 0.18 parts |

### COMPARATIVE EXAMPLE 4

An adhesive preparation was produced in the same manner as in Example 3, except that the backing used was the same as that used in Comparative Example 1.

### EXAMPLE 4

A toluene solution of a drug-containing pressure-sensitive adhesive was prepared according to the following formulation. The solution obtained was applied to a poly(ethylene terephthalate) liner (75 µm) which had undergone a silicone releasant treatment, in such an amount as to result in a thickness after drying of 80 µm. This coated liner was dried at 100°C for 3 minutes in a hot-air circulating drying oven. Thus, a pressure-sensitive adhesive layer was obtained. The same backing as in Example 1 was press-bound on its film side to the pressure-sensitive adhesive layer with a rubber roller to produce an adhesive preparation according to the invention.

### Formulation

| Pressure-sensitive adhesive ingredient: | | |
|---|---|---|
| | Polyisobutylene (viscosity-average molecular weight, about 4,000,000) | 18 parts |
| | Polyisobutylene (viscosity-average molecular weight, about 55,000) | 18 parts |

| Tackifier ingredient: | | |
|---|---|---|
| | Alicyclic saturated hydrocarbon resin | 24 parts |

| Liquid ingredient: | | |
|---|---|---|
| | Isopropyl myristate | 25 parts |

| Drug: | | |
|---|---|---|
| | Emedastine (having four tertiary amine structures) | 15 parts |

### COMPARATIVE EXAMPLE 5

An adhesive preparation was produced in the same manner as in Example 4, except that the backing used was the same as that used in Comparative Example 1.

### EXAMPLE 5

A hexane solution of a drug-containing pressure-sensitive adhesive was prepared according to the following formulation. The solution obtained was applied to a poly(ethylene terephthalate) liner (75 µm) which had undergone a fluorochemical releasant treatment, in such an amount as to result in a thickness after drying of 60 µm. This coated liner was dried at 80°C for 5 minutes in a hot-air circulating drying oven. Thus, a pressure-sensitive adhesive layer was obtained. The same backing as in Example 4 was press-bound on its nonwoven-fabric side to the pressure-sensitive adhesive layer with a rubber roller to produce an adhesive preparation according to the invention.

### Formulation

| Pressure-sensitive adhesive ingredient: | | |
|---|---|---|
| | Silicone pressure-sensitive adhesive (condensate of polydimethylsiloxane with silicone resin) | 66 parts |

| Liquid ingredient: | | |
|---|---|---|
| | Isopropyl myristate | 2 parts |

| Liquid ingredient: | | |
|---|---|---|
| | Oleic acid | 2 parts |

| Drug: | | |
|---|---|---|
| | Salbutamol (having one secondary amine structure) | 30 parts |

### COMPARATIVE EXAMPLE 6

An adhesive preparation was produced in the same manner as in Example 5, except that the backing used was the same as that used in Comparative Example 1.

### EXAMPLE 6

A toluene solution of a drug-containing pressure-sensitive adhesive was prepared according to the following formulation. The solution obtained was applied to a poly(ethylene terephthalate) liner (75 µm) which had undergone a silicone releasant treatment, in such an amount as to result in a thickness after drying of 80 µm. This coated liner was dried at 100°C for 3 minutes in a hot-air circulating drying oven. Thus, a pressure-sensitive adhesive layer was obtained. The same backing as in Example 1 was press-bound on its nonwoven-fabric side to the pressure-sensitive adhesive layer with a rubber roller to produce an adhesive preparation according to the invention.

### Formulation

| Pressure-sensitive adhesive ingredient: | | |
|---|---|---|
| | Polyisobutylene (viscosity-average molecular weight, about 4,000,000) | 18 parts |
| | Polyisobutylene (viscosity-average molecular weight, about 55,000) | 18 parts |

| Tackifier ingredient: | | |
|---|---|---|
| | Alicyclic saturated hydrocarbon resin | 24 parts |

| Liquid ingredient: | | |
|---|---|---|
| | Isopropyl myristate | 30 parts |

| Drug: | | |
|---|---|---|
| | Propranolol (having one secondary amine structure) | 10 parts |

### COMPARATIVE EXAMPLE 7

An adhesive preparation was produced in the same manner as in Example 6, except that the backing used was the same as that used in Comparative Example 1.

### EXAMPLE 7

An adhesive preparation according to the invention was produced in the same manner as in Example 3, except that the backing used was the backing used in Example 1 in which the thickness of the poly(ethylene terephthalate) film was 4.5 µm.

### TEST EXAMPLE

### 1. Wear Test

Samples having a size of 5 cm × 5 cm were punched out of each of the adhesive preparations produced in Example 2, Comparative Example 2, and Comparative Example 3. These samples were applied to a central part of the upper chest and an upper chest part near an armpit of each of six volunteers. The volunteers wore the samples for 24 hours and evaluated the samples for wear feeling according to the following criteria.
Stiff Feeling
   1: satisfactory wearing, with almost no stiff feeling
   2: slight stiff feeling is given
   3: considerable stiff feeling is given
Stretched Feeling
   1: satisfactory wearing, with almost no stretched feeling
   2: slight stretched feeling is given
   3: considerable stretched feeling is given

**Table 1**

| Results of Wear Test | | | |
|---|---|---|---|
| | | Stiff feeling | Stretched feeling |
| Example 2 | Central part of upper chest | 1 | 1 |
| | Upper chest part near armpit | 1 | 1 |
| Comparative Example 2 | Central part of upper chest | 1 | 2 |
| | Upper chest part near armpit | 1 | 1 |
| Comparative Example 3 | Central part of upper chest | 3 | 3 |
| | Upper chest part near armpit | 3 | 3 |

The adhesive preparations of Example 2 and Comparative Example 2 gave almost no stiff feeling because they employed a backing composed of a thin film and a nonwoven fabric having a low basis weight. The adhesive preparation of Example 2 gave almost no stretched feeling during wear on the active part. The adhesive preparation of Comparative Example 3 gave both a considerable stiff feeling and a considerable stretched feeling and was impracticable.

### 2. Light Transmittance Test

With respect to each of Examples 3 to 7 and Comparative Examples 4 to 7, the liner was stripped off and this adhesive preparation was examined with a spectrophotometer for light transmittance at a wavelength of 610 nm, with the pressure-sensitive adhesive layer side faced the light-receiving part of the spectrophotometer.

**Table 2**

| Results of Transmittance Test | |
|---|---|
| | Transmittance (610 nm) |
| Example 3 | 56% |
| Example 4 | 55% |
| Example 5 | 55% |
| Example 6 | 57% |
| Example 7 | 59% |
| Comparative Example 4 | 45% |
| Comparative Example 5 | 44% |
| Comparative Example 6 | 47% |
| Comparative Example 7 | 45% |

The adhesive preparations of Examples 3 to 7 and Comparative Examples 4 to 7 were examined for transmittance at 610 nm. The adhesive preparations employing a backing produced by thermal fusion bonding had a higher light transmittance and higher transparency.

### 3. Backing Film Adhesion Strength Test

With respect to Examples 3 to 7 and Comparative Examples 4 to 7, the adhesive preparations were packed with an aluminum/polyacrylonitrile laminate packaging material and stored at 50°C for 2 months. Thereafter, each adhesive preparation was cut into a 16-mm square and applied to a bakelite plate after liner stripping. A finger was slid on the film side of this adhesive preparation while strongly pushing the finger against the film. The adhesive preparation was examined for film separation upon this sliding.

**Table 3**

| Results of Backing Film Adhesion Strength Test | |
|---|---|
| | Film Adhesion Strength (2-month storage at 50°C) |
| Example 3 | no separation occurred |
| Example 4 | no separation occurred |
| Example 5 | no separation occurred |
| Example 6 | no separation occurred |
| Example 7 | no separation occurred |
| Comparative Example 4 | separation occurred |
| Comparative Example 5 | separation occurred |
| Comparative Example 6 | separation occurred |
| Comparative Example 7 | separation occurred |

After the 2-month storage at 50°C, the adhesion strengths of the backing films in Examples 3 to 7 and Comparative Examples 4 to 7 were as follows. In the Examples, which employed a backing produced by thermal fusion bonding, none of the adhesive preparations suffered film separation. In contrast, in the Comparative Examples, which employed a backing produced with a binder, the adhesive preparations suffered film separation.

### 4. Drug Storage Stability Test

With respect to Example 6, Example 7, and Comparative Example 7, the adhesive preparations were packed with an aluminum/polyacrylonitrile laminate packaging material and stored at 50°C for 1 month. The drug content resulting from this storage was compared with the initial drug content to determine drug retention in percentage. Each drug content was determined by dissolving/extracting the adhesive preparation with an organic solvent and determining the amount of the drug present in the solution by HPLC.

**Table 4**

| Results of Drug Storage Stability Test | |
|---|---|
| | Drug Retention (1-month storage at 50°C) |
| Example 6 | 98.2 ± 0.2% |
| Example 7 | 99.3 ± 0.6% |
| Comparative Example 7 | 73.1 ± 0.7% |

The adhesive preparation of Comparative Example 7, which employed a backing having a binder, showed reduced drug storage stability. On the other hand, the adhesive preparation of Example 7 showed exceedingly satisfactory drug stability because it employed a backing obtained by thermally fusion-bonding a poly(ethylene terephthalate) film to a nonwoven poly(ethylene terephthalate) fabric.

### 5. Anchoring Test

With respect to each of Example 6, Example 7, and Comparative Example 7, the backing was press-bound by rolling a 2-kg rubber roller thereon forward and backward once. This adhesive preparation was allowed to stand at room temperature for a certain time period, and two samples having a size of 3 cm × 5 cm were punched out of the adhesive preparation while taking care not to apply a pressure thereto. After the liner was stripped off, an end part of each adhesive preparation was folded back in a width of about 5 mm to form a handling part. The exposed surfaces of the pressure-sensitive adhesive layers were lightly bound to each other, and this specimen was allowed to stand for 10 minutes. This specimen was held by the handling parts to peel the backings from each other. Anchoring to the backings was evaluated based on the following criteria.
1: the pressure-sensitive adhesive layers could not be peeled from each other
2: part of the pressure-sensitive adhesive layers remained on one backing
3: most of the pressure-sensitive adhesive layers remained on one backing
4: the pressure-sensitive adhesive layers partly remained on the liners
5: the pressure-sensitive adhesive layers mostly remained on the liners

**Table 5**

| Results of Anchoring Test | | | | |
|---|---|---|---|---|
| | Anchoring Test | | | |
| | After 1 h | After 3 h | After 6 h | After 12 h |
| Example 6 | 2 | 1 | 1 | 1 |
| Example 7 | 2 | 1 | 1 | 1 |
| Comparative Example 7 | 5 | 4 | 2 | 1 |

The adhesive preparations of Example 6 and Example 7 came to have sufficient anchoring in a short time period. In contrast, the adhesive preparation of Comparative Example 7 required 12 hours for obtaining anchoring.

## Claims

1. An adhesive preparation comprising:
a backing; and
a pressure-sensitive adhesive layer which contains a drug and is laminated on at least one side of the backing,
wherein the backing comprises a polyester film having a thickness of 0.5 to 6.0 µm, and a polyester nonwoven fabric directly bound to said film without using a binder,
wherein the drug has at least one primary to tertiary amine structure within the molecule thereof.

2. The adhesive preparation according to claim 1, wherein the pressure-sensitive adhesive layer is directly or indirectly laminated on said nonwoven-fabric side of the backing.

3. The adhesive preparation according to claim 1, wherein a polyester fiber in said nonwoven fabric is directly bound to a part of the polyester film to which said nonwoven fabric is bound.

4. The adhesive preparation according to claim 1, wherein said nonwoven fabric has a basis weight of 5 to 40 g/m².

5. The adhesive preparation according to claim 1, wherein said film and said nonwoven fabric are fusion-bonded to each other.

6. The adhesive preparation according to claim 1, wherein the pressure-sensitive adhesive layer is laminated on said film side of the backing.

## Patentansprüche

1. Klebstoffzubereitung, umfassend:
eine Trägerschicht; und
eine druckempfindliche Klebstoffschicht, die ein Arzneimittel enthält, und die auf mindestens eine Seite der Trägerschicht laminiert ist,
wobei die Trägerschicht einen Polyesterfilm, der eine Dicke von 0,5 bis 6,0 µm besitzt, und einen Polyestervliesstoff, der direkt an besagten Film ohne Verwendung eines Binders gebunden ist, umfasst,
wobei das Arzneimittel mindestens eine primäre bis tertiäre Aminstruktur innerhalb des Moleküls davon besitzt.

2. Klebstoffzubereitung gemäß Anspruch 1, wobei die druckempfindliche Klebstoffschicht direkt oder indirekt auf besagter Vliesstoffseite der Trägerschicht laminiert ist.

3. Klebstoffzubereitung gemäß Anspruch 1, wobei eine Polyesterfaser in besagtem Vliesstoff direkt an einen Teil des Polyesterfilms, an den besagter Vliesstoff gebunden ist, gebunden ist.

4. Klebstoffzubereitung gemäß Anspruch 1, wobei besagter Vliesstoff ein Basisgewicht von 5 bis 40 g/m² besitzt.

5. Klebstoffzubereitung gemäß Anspruch 1, wobei besagter Film und besagter Vliesstoff aneinander fusionsgebunden sind.

6. Klebstoffzubereitung gemäß Anspruch 1, wobei die druckempfindliche Klebstoffschicht auf besagter Filmseite der Trägerschicht laminiert ist.

## Revendications

1. Préparation adhésive comprenant :
un support ; et
une couche adhésive sensible à la pression qui contient un médicament et qui est stratifiée sur au moins un côté du support,
dans laquelle le support comprend un film de polyester présentant une épaisseur de 0,5 à 6,0 µm et un non-tissé de polyester directement lié audit film sans utiliser un liant,
dans laquelle le médicament présente au moins une structure d'amine primaire à tertiaire à l'intérieur de sa molécule.

2. Préparation adhésive selon la revendication 1, dans laquelle la couche adhésive sensible à la pression est directement ou indirectement stratifiée sur ledit côté de non-tissé du support.

3. Préparation adhésive selon la revendication 1, dans laquelle une fibre de polyester dans ledit non-tissé est directement liée à une partie du film de polyester à laquelle ledit non-tissé est lié.

4. Préparation adhésive selon la revendication 1, dans laquelle ledit non-tissé présente un poids de base de 5 à 40 g/m².

5. Préparation adhésive selon la revendication 1, dans laquelle ledit film et ledit non-tissé sont liés l'un à l'autre par fusion.

6. Préparation adhésive selon la revendication 1, dans laquelle la couche adhésive sensible à la pression est stratifiée sur ledit côté de film du support.
